# EUROPEAN PATENT APPLICATION

(11) **EP 2 757 087 A1**
(43) Date of publication of application: **23.07.2014**
(21) Application number: 12827812.4
(22) Date of filing: 28.03.2012
(51) Int. Cl.: C07C 67/28, C07C 69/587, C07C 31/22, C10G 3/00

(54) **METHOD FOR PRODUCING BIODIESEL**

(30) Priority: 26.08.2011 KR 20110085789; 27.03.2012 KR 20120031244
(71) Applicant: Research Institute Of Industrial Science&Technology, Kyungsangbuk-do 790-330 (KR)
(72) Inventor: KWON, Eilhann, Seoul 156-861 (KR); SEO, Jaegun, Gyeongsan-si Gyeongsangbuk-do 712-100 (KR); YI, Haakrho, Gwangyang-si Jeollanam-do 545-764 (KR); PARK, Jongseok, Goyang-si Gyeonggi-do 412-500 (KR); HWANGBO, Jun Kwon, Gwangyang-si Jeollanam-do 545-786 (KR)
(74) Representative: Potter Clarkson LLP
(86) International application number: PCT/KR2012/002268
(87) International publication number: WO 2013/032090

(57) **Abstract**

Disclosed is a method for producing biodiesel, the method including generating fatty acid alkyl esters and glycerol by a transesterification reaction of oil and an aliphatic alcohol in a presence of a refractory porous material. The method may have a high reaction rate,and a high FAME conversion, and the method may be performed by a continuous process. Further, according to the method, high-purity fatty acid alkyl esters and glycerol may be produced regardless of free fatty acids (FFAs). Further, since a catalyst is not used in the method, the overall process time and cost may be saved, and the method is environmetally friendly.

## Description

### [Technical Field]

The present invention relates to a method for converting oils into biodiesel (fatty acid methyl esters, FAMEs) through a transesterification reaction.

### [Background Art]

Currently, among biodiesel producing processes, only a transesterification reaction has been commercialized.

In such a method of conversion into biodiesel (fatty acid methyl esters, FAMEs) through a transesterification reaction, triglyceride is a basic component of edible/non-edible oils that are used.

While FAMEs and glycerin are generated from triglyceride through the transesterification reaction, its reaction rate is very slow. Therefore, acidic catalysts and/or basic catalysts are used to increase the rate of the transesterification reaction in the commercialized biodiesel producing process.

It is known that the reaction time to obtain a FAME conversion of 95% through a transesterification reaction is not less than 30 hours for an acidic catalyst and not less than 2 hours for a basic catalyst. Naturally, the use of the basic catalyst may be more favorable, but the fatty acids separated from triglyceride, that is, free fatty acids (FFAs) are problematic. Due to these free fatty acids, oils having a high acid value (1 or higher) cause a saponification reaction in a transesterification reaction using a basic catalyst. The free fatty acids are relatively easily generated from oils by an external stimulus such as heat, solar light, or oxidation. Therefore, it may be considered that free fatty acids are present in all the oils used in the biodiesel producing process.

Currently, the commercialized biodiesel conversion process includes a pretreatment process using an acidic catalyst (H₂SO₄) and a main treatment process using a basic catalyst (KOH, NaOH).

The pretreatment process and the main treatment process require long reaction times of at least 30 hours and at least 2 hours, respectively. Since these pretreatment and main treatment processes are batch reactions, they are difficult to apply in the mass production of FAMEs.

Moreover, the commercialized biodiesel conversion process necessarily entails a washing process using warm water due to the use of the acidic catalyst and the basic catalyst. However, the washing process causes a large amount of waste water and a loss of oils, which are reactants, resulting in remarkably deteriorating the overall process yield.

Moreover, when the above catalysts are used to produce mass biodiesel in the commercialized biodiesel reaction process, a washing process and a purification and separation process take much time and cause a large amount of waste water. A natural purification process not using a centrifuge takes at least one hour or usually two or more hours. Here, biodiesel may be washed out during the washing process and the purification and separation process, resulting in reducing a biodiesel production yield.

In order to solve the drawbacks due to the use of the above catalysts, a supercritical transesterification process has been studied that methanol or ethanol and an oil component are allowed to react at 120 to 150°C at the high pressure conditions of 50 to 200 bar, thereby directly obtaining fatty acid alkyl esters.

Although the supercritical transesterification process may collect fatty acid alkyl esters generated, a large amount of alcohol such as methanol or ethanol, however, is used such that the ratio of alcohols to oils is approximately 20:1. Moreover, in the supercritical transesterification process, continuous processing is difficult and production costs increase due to high pressure conditions. Accordingly, the supercritical transesterification process corresponds to merely R&D-level technology until now. Particularly, since the supercritical transesterification process has lower energy efficiency due to the introduction of excessive ethanol, commercialization of the supercritical transesterification process for biodiesel production still seems to have a long way to go.

The above information disclosed in this Background section is only for enhancement of understanding of the background of the invention and therefore it may contain information that does not form the prior art that is already known in this country to a person of ordinary skill in the art.

### [DISCLOSURE]

### [Technical Problem]

An embodiment of the present invention may provide a method for producing biodiesel having advantages of realizing mass production by employing a continuous process.

An embodiment of the present invention may provide a method for producing biodiesel having advantages of obtaining high-purity fatty acid alkyl esters and glycerol regardless of the content of fatty acid contained in oils.

An embodiment of the present invention may provide a method for producing biodiesel having advantages of preventing the generation of waste water and the drop in the yield of biodiesel during a washing process for removing an acidic catalyst or a basic catalyst.

An embodiment of the present invention may provide a method for producing biodiesel having advantages of being environmentally friendly and achieving a significant energy saving effect.

### [Technical Solution]

An embodiment of the present invention started to be made from the thinking that a catalyst-free transesterification reaction may be induced by supplying the heat energy higher than activation energy necessary for the reaction to reactants. In other words, it seems that the temperature may be a main driving force for the transesterification reaction.

An embodiment of the present invention provides a method for producing biodiesel, the method including generating fatty acid alkyl esters and glycerol by a transesterification reaction of oils and aliphatic alcohols in the presence of a porous material.

The temperature of the transesterification reaction may be controlled such that the transesterification reaction proceeds in the presence of a porous material, by raising energy levels of the oils and the aliphatic alcohols through a supply of heat energy.

The temperature of the transesterification reaction may be controlled such that the fatty acid alkyl esters are generated in a gas phase and thermal cracking of the oils is not generated.

The temperature of the transesterification reaction may be 350 to 500°C.

The retention time of the transesterification reaction may be 0.1 to 5 minutes.

The weight ratio of the oils to the alcohols may be 1:0.05 to 1:1.

The transesterification reaction may be performed in the gas atmosphere of at least one of nitrogen, argon, or carbon dioxide.

The pressure of the transesterification reaction may be 10 mmHg to 10 atm.

The transesterification reaction may be performed in a heterogeneous phase reaction.

The transesterification reaction may be performed in a continuous process.

The porous material may be at least one of alumina (Al₂O₃), zeolite, activated carbon, charcoal, or silica.

The porous material may include a mesoporous material, a macroporous material, or both thereof.

An embodiment of the present invention provides a method for producing biodiesel, the method including: inducing a transesterification reaction between oils and aliphatic alcohols by continuously supplying the oils and the aliphatic alcohols into a reactor charged with a refractory porous material; and collecting gas-phase fatty acid alkyl esters and glycerol, which are generated by the transesterification reaction.

In the inducing of the transesterification reaction, a purge gas may be continuously supplied together with the oils and the aliphatic alcohols, the purge gas being at least one of nitrogen, argon, or carbon dioxide.

### [Advantageous Effects]

According to an embodiment of the present invention, biodiesel may be effectively mass-produced in a continuous process by a transesterification reaction of oils and aliphatic alcohols in the presence of a porous material. Further, since an acidic catalyst or a basic catalyst is not used, harmful effects due to the use of the acidic catalyst and the basic catalyst may be prevented.

Particularly, since the method for producing biodiesel without catalysts according to an embodiment of the present invention eliminates a washing process, the method may be environmentally friendly and have an energy saving effect producing high-purity fatty acid alky esters and glycerol. Further, according to an embodiment of the present invention, waste water treatments due to the washing process, which is caused by the use of catalysts, is not performed, thereby saving the total process time and cost for producing biodiesel and producing regenerative fuels through a more environmentally friendly process.

### [Description of the Drawings]

FIG. 1 is a table showing a fatty acid profile of oils according to an embodiment of the present invention;
FIG. 2 is a schematic view showing a mechanism of a transesterification reaction according to an embodiment of the present invention;
FIG. 3 is a schematic view showing a mechanism of a transesterification reaction using MeOH according to an embodiment of the present invention;
FIGS. 4A to 4C are graphs showing pore distributions of activated alumina, cordierite, and charcoal, respectively;
FIG. 5 is an image showing biodiesel (FAMEs, in the left flask) and glycerin-separated biodiesel (FAMEs, in the right flask), which were obtained through a catalyst-free continuous type transesterification reaction according to an embodiment of the present invention;
FIG. 6 is a graph showing thermo-gram and differential thermo-gram (DTG) of soybean oil according to an embodiment of the present invention;
FIG. 7 is a graph showing the FAME conversion depending on control of the temperature according to an embodiment of the present invention;
FIG. 8 is a table showing boiling points of FAMEs according to an embodiment of the present invention;
FIG. 9 is a graph showinig the FAME conversion depending on control of the mixture ratio of MeOH and oils according to an embodiment of the present invention;
FIG. 10 is an SEM image of charcoal as a porous material according to an embodiment of the present invention;
FIG. 11 is a graph showing the FAME conversion when carbon dioxide or nitrogen was used;
FIG. 12 is a graph showing the FAME conversion for an example in which 100% of fatty acid was used in the reaction according to an embodiment of the present invention;
FIG. 13 is a graph showing the FAME conversion when charcoal was used according to an embodiment of the present invention (Example 12);
FIG. 14 is a graph showing the FAME conversion when ethanol (EtOH) was used according to an embodiment of the present invention (Example 14);
FIG. 15 is a graph showing the FAME conversion using a thermo-chemical method of the related art (Comparative Example 3); and
FIG. 16 shows a mass chromatogram for products which were obtianed after a transesterification reaction was performed using beef tallow and lard in Example 16.

### [Mode for Invention]

According to an embodiment of the present invention, there is provided a method for producing biodiesel, the method including generating fatty acid alkyl esters and glycerol by a transesterification reaction of oils and aliphatic alcohols in the presence of a porous material.

Hereinafter, methods for producing biodiesel, in which oils are converted into fatty acid alkyl esters through a transesterification reaction according to embodiments of the present invention, will be described in more detail. However, these embodiments are provided merely for illustrating the present invention, and it will be apparent to those skilled in the art that the scope of the present invention is not limited by these embodiments and various modifications of the embodiments may be made within the scope and technical range of the present invention.

Further, unless particularly mentioned herein, the term "including" or "containing" refers to including some elements (or components) without any limitation, and should not be construed as excluding addition of other elements (or components).

In the procedure of repeated researches about the production of biodiesel from oils through a transesterification reaction, the present inventors found that the use of a porous material without the introduction of a separate catalyst may lead to the production of high-purity fatty acid alkyl esters and glycerol in a continuous type, not in a bath type, regardless of the presence or absence, the content, or the kinds of free fatty acids (FFAs) contained in oils, and then completed an embodiment of the present invention.

According to an embodiment of the present invention, a transesterification reaction is induced by a thermo-chemical conversion method. According to an embodiment of the present invention, neither liquid nor solid catalysts are needed and high-pressure supercritical conditions are not required.

The present inventors determined that the reaction temperature may be a main driving force for a transesterification reaction, and thought that the activation energy of the transesterification reaction may be sufficiently reached by the supply of heat energy. The present inventors noticed that the activation energy of the transesterification reaction is lower than those of other catalytic reactions such as methane steam reforming and the like.

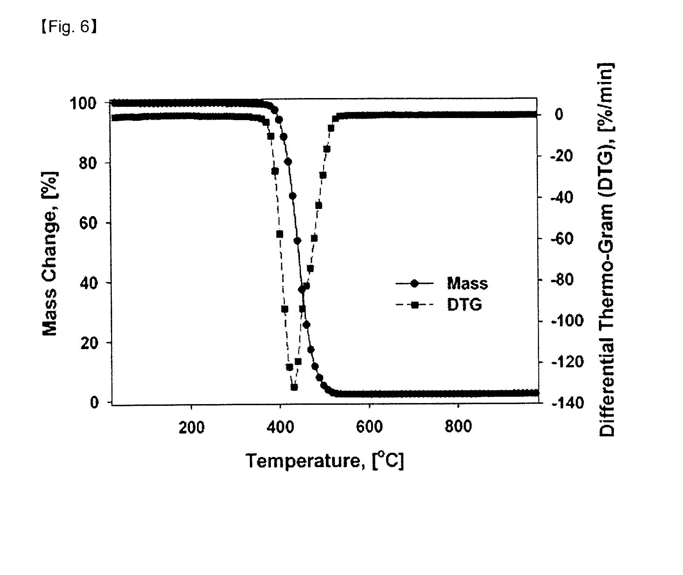

The present inventors determined that a catalyst-free transesterification reaction is possible through a heterogeneous thermo-chemical process. Here, the heterogeneous thermo-chemical process includes reactions occurring between liquid-phase oils and gas-phase alcohols at a high temperature. However, the allowable temperature range for a catalyst-free transesterification reaction is uncertain. Therefore, thermo-gravimetric analysis (TGA) was performed on the oils to investigate the allowable temperature range. The results are shown in FIG. 6. The thermo-gravimetric analysis associated with FIG. 6 will be later described in detail.

The present inventors tried a catalyst-free transesterification reaction in the temperature range which was investigate through the thermo-gravimetric analysis, but aliphatic alkyl esters were not significantly converted. From the judgment of the present inventors, the reason is that the contact time between the liquid-phase oils and the gas-phase alcohols is short. Thus, the present inventors used a porous material having tortuosity and adsorption capability in the catalyst-free transesterification reaction in order to increase the contact time of the heterogeneous phase reactants.

According to one embodiment of the present invention, the transesterification reaction is induced by raising energy levels of the reactants through the supply of a heat source and using a porous material, unlike a transesterification reaction using a catalyst in the related art. Therefore, according to an embodiment of the present invention, biodiesel may be obtained even when only alcohols (MeOH, EtOH, etc.) and oils are used as reactants without an additional catalyst.

According to an embodiment of the present invention, the oils include microalgae oils as well as edible and non-edible oils. The oils include all of oils or fats including triglyceride, and include even their free fatty acids (FFAs). Particularly, the oils may include triglyceride represented by Chemical Formula 1. 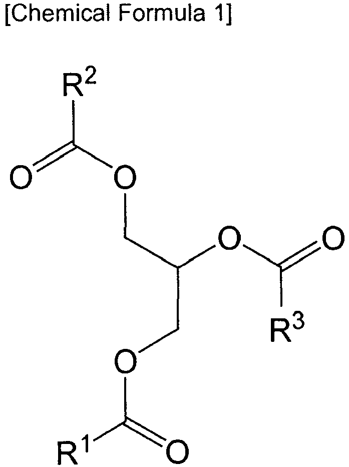 wherein,
R¹, R², and R³ are the same as or different from each other, and each may be a C₄-C₃₈ aliphatic hydrocarbon group, preferably a C₄-C₂₄ aliphatic hydrocarbon group, or more preferably a C₁₂-C₂₀ aliphatic hydrocarbon group. The number of carbon atoms of the aliphatic hydrocarbon group in the triglyceride may be optimized to be similar to that in the general diesel.

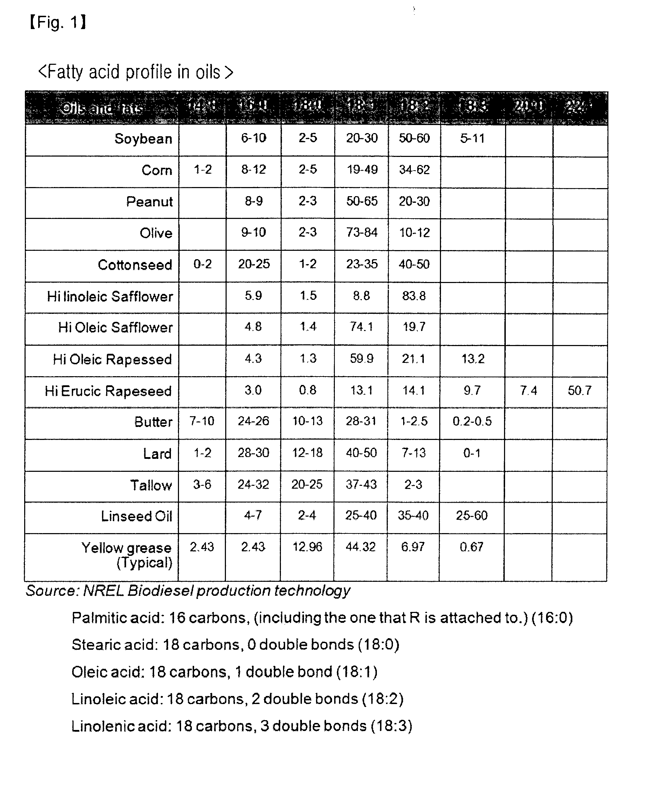

Physical and chemical properties (viscosity, molecular weight, boiling point, etc.) of the oils may be varied depending on the kinds of fatty acids (FAs) present in triglyceride, in other words, the kinds of substituents R¹, R², R³ or the like in Chemical Formula 1. The fatty acid profile for representative components of the oils are as exemplified in FIG. 1.

In addition, examples of the oils may be ones including triglyceride represented by Chemical Formula 2 below.

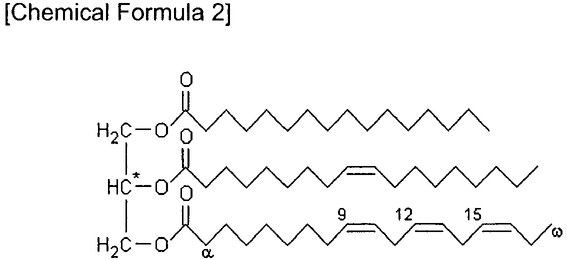

According to an embodiment of the present invention, the oils may include small amounts of other impurities or water. Particularly, according to an embodiment of the present invention, a transesterification reaction may be effectively performed even when the reactants contain water. For example, even while the used cooking oil may contain a large amount of water or even the non-edible cooking oil may contain a slight amount of water, these oils, as they are, may be used in the transesterification reaction according to an embodiment of the present invention, without a moisture removal process. However, for some kinds of oils, a content of water may be minimized within an appropriate range in order to attain more improved process efficiency.

Examples of the oils according to an embodiment of the present invention may include palm oil, soybean oil, rapeseed oil, corn oil, rape oil, sunflower oil, safflower oil, cottonseed oil, sesame oil, perilla oil, rice bran oil, palm kernel oil, camellia oil, castor oil, olive oil, coconut oil, almond oil, jatropha oil, diatom, sewage sludge, microalgae, beef tallow, lard, sun, fish oil, whale oil, tuna oil, and the like. In addition, one or more of these oils may be used in a mixture thereof, and their waste oils may be also used.

According to an embodiment of the present invention, aliphatic alcohols or primary alcohols as a reactant may be used together with the oils. For example, C₁-C₁₂ aliphatic alcohols may be used. Examples of these aliphatic alcohols may include methanol, ethanol, propanol, butanol, pentanol, and the like. One or more of these alcohols may be used in a mixture thereof.

Of the alcohols, methanol and ethanol may be preferable in the view of costs. In consideration of reactivity, methanol may be more suitable than ethanol. In the case of methanol (MeOH), the reactivity in the transesterification reaction may become better due to reactivity and steric factors of methanol. In addition, alcohols having many carbon atoms may be used to increase the number of carbon atoms in the generated FAMEs. Particularly, the use of various kinds of alcohols having different carbon atoms may variously change physical properties of the generated FAMEs.

In the reaction of the oils and the alcohols, the weight ratio of the oils to the alcohols may be within the range of 1:0.1 to 1:1, preferably 1:0.15 to 1:1, and more preferably 1:0.2 to 1:1. In some cases, the weight ration of the oils to the alcohols may be 1:0.05. In addition, the alcohols may be used in a content of 10 parts by weight or more, preferably 15 parts by weight or more, and more preferably 20 parts by weight, based on 100 parts by weigh of the oils. In the aspect of stoichiometry, the content of the alcohols is preferably 10 parts by weight or more based on 100 parts by weight of the oils. Alternatively, a large amount of alcohols may react with the oils, but in the aspect of energy efficiency, the alcohols may react with the oils while the use amount of alcohols is minimized. Alternatively, more than 100 parts by weight of the alcohols may be used based on 100 parts by weight of oils. However, the present inventors found that the excess use of alcohols merely increased the process cost but did not obtain significant improvement in effects.

When the method of using an acidic catalyst or a basic catalyst or the supercritical transesterification method is applied in the related art, the use of about 10 parts by weight of alcohols based on 100 parts by weight of oils never induced a transesterification reaction for generating biodiesel or required a reaction time of at least two hours. However, according to an embodiment of the present invention, even when using a small amount of alcohols as described above, a conversion of approximately 90% or higher, 95% or higher for appropriate reaction conditions, and 98% or higher for more appropriate reaction conditions may be obtained in a short time of 5 minutes or less, or approximately less than 1 minute based on experimental results confirmable by the present inventors. Particularly, an embodiment of the present invention is characterized by using a remarkably small amount of alcohols as compared with the biodiesel conversion process of the related art.

An embodiment of the present invention is characterized by allowing a catalyst-free transesterification reaction using the oils and the aliphatic alcohols in the presence of a porous material, instead of using an acidic catalyst or a basic catalyst like in the related art. According to an embodiment of the present invention, the porous material does not function as a catalyst, which is clearly differentiated from a solid-phase catalyst.

The porous material according to an embodiment of the present invention maintains its porosity even in a thermo-chemical conversion procedure, in other words, a high-temperature transesterification reaction. For example, the porous material may be not thermally decomposed within the reaction temperature range according to an embodiment of the present invention. In this regard, it is explained that that the porous material according to an embodiment of the present invention is refractory or the porous material according to an embodiment of the present invention is called a refractory material.

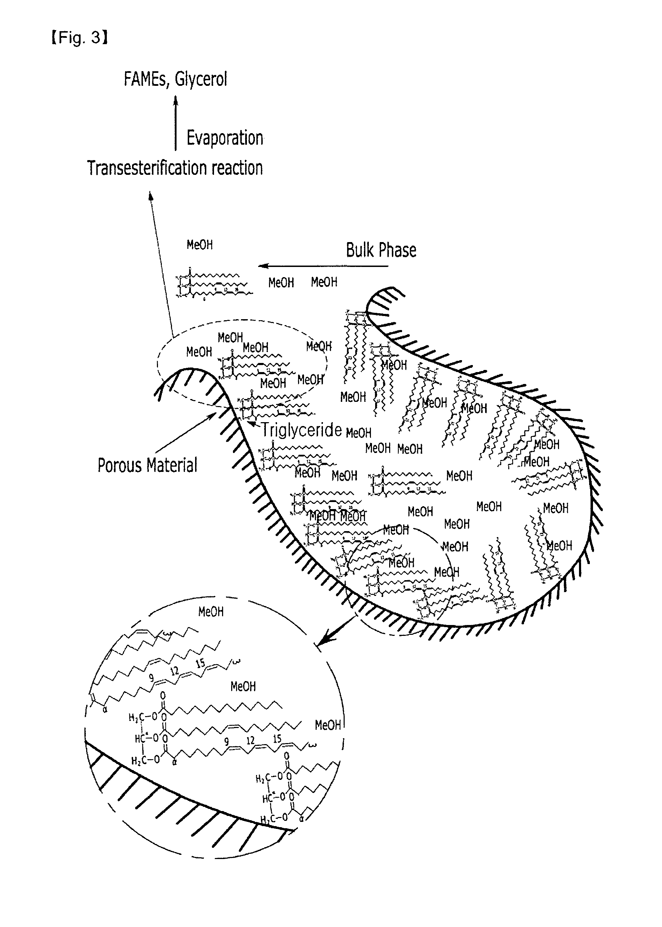

The refractory porous material according to an embodiment of the present invention may receive an activated reactant, and may be any porous material having pores in which the reaction between the oils and the alcohols may be effectively performed. Alcohols evaporated due to the high temperature have an increased kinetic energy, and thus collide with the oils adsorbed on the pores. As a result, a transesterification reaction may promptly proceed. The reaction mechanism will be described in more detail with reference to FIG. 3.

The refractory porous material according to an embodiment of the present invention may have various pore sizes and pore distributions in the conditions allowing evaporation and adsorption of reactants. For example, as the refractory porous material, a mesoporous material (pore diameter: not smaller than 1 nm but smaller than 50 nm) or a macroporous material (pore diameter: not smaller than 50 nm but not larger than 500 *µ*m) may be used. However, in the view of optimization of the reactor size and improvement in process efficiency, the refractory porous material may have pores with an average diameter of not smaller than 1 nm or not smaller than 1 nm but not larger than 500 *µ*m, preferably not smaller than 1.5 nm, and more preferably not smaller than 2 nm.

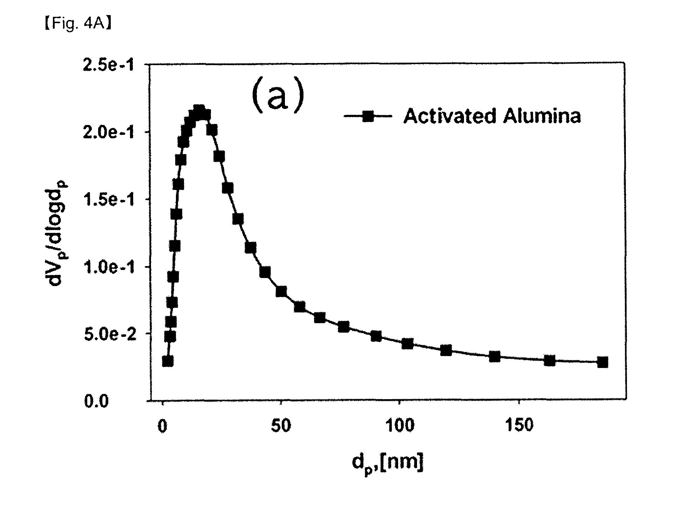

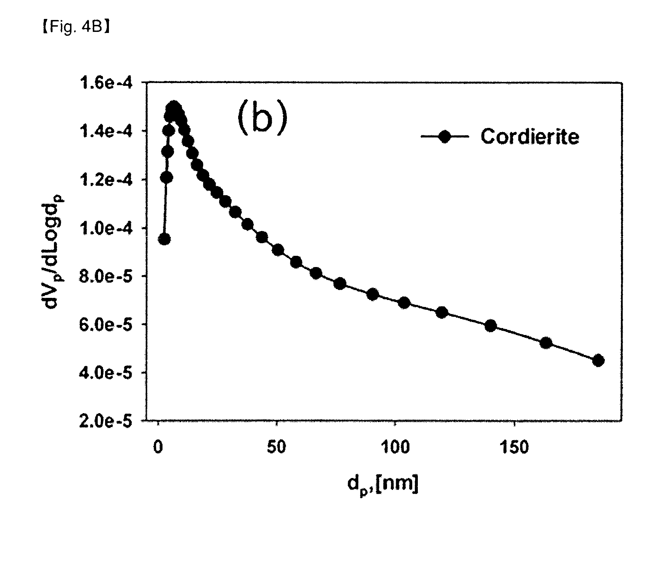

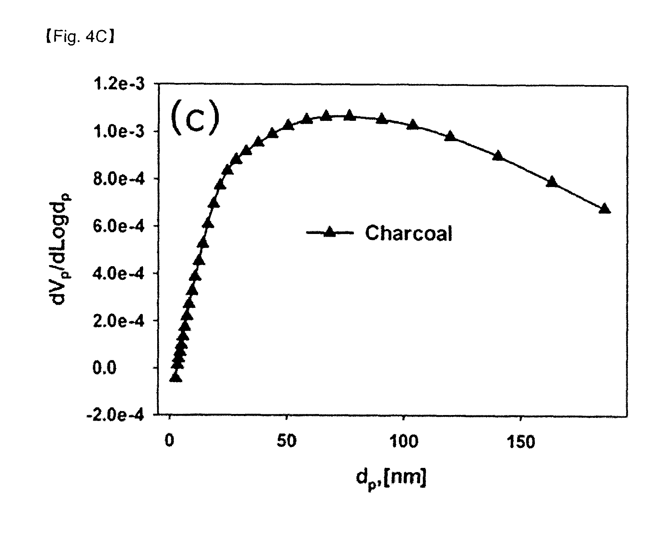

For the understanding of the size or shape of pores of the porous material according to an embodiment of the present invention, some examples are shown in FIGS. 4A to 4C. FIG. 4A shows a pore distribution of activated alumina, and FIGS. 4B and 4C show pore distributions of cordierite and charcoal, respectively. As shown in FIGS. 4A to 4C, it is determined that the dominant pore size, in other words, the pore size, at which the largest density or peak is shown in each pore distribution curve, is approximately 10 nm or larger.

As above, the present inventors illuminated that the use of the porous material was considered in order to increase the contact time between the oils and the alcohols in the reaction conditions according to an embodiment of the present invention. Since the average molecular size of triglyceride is approximately 2 nm, it is determined that pores having a size of at least 2 nm need to be dominant in the porous material according to an embodiment of the present invention, in order to allow triglyceride to be adsorbed or contained in the pores and allow a transesterification reaction between the oils and the alcohols to smoothly proceed in the pores.

It is difficult to clearly define the porous material according to an embodiment of the present invention in a qualitative manner by using the pore size. However, based on experiment results so far, the present inventors confirmed that a porous material, which may be generally called a mesoporous material or a macroporous material, may be used as the porous material according to an embodiment of the present invention. Considering FIGS. 4A to 4C, a porous material having a dominant pore size of 10 nm or larger may be preferable.

Since a carrier for high-temperature (higher than 500°C), which is generally used for a catalytic reaction or the like, is subjected to sintering treatment, its pores may be closed or smaller during the sintering treatment. In an embodiment of the present invention, when this conventional carrier is used, the amount of mesoporous materials is relatively decreased, and thus a more amount of filler is needed and the size of the reactor may be excessively enlarged. Particularly, in the case of the sintered carrier of the related art, in other words, the sintered carrier of a microporous material having nano-sized pores, an effective reaction of an embodiment of the present invention may be not exhibited even when a more amount of filler is introduced into the carrier. In this regard, according to an embodiment of the present invention, a refractory porous material in which mesopores account for 80% or more of the overall pores may be used.

According to an embodiment of the present invention, any refractory porous material known until now may be used, and even a brick having pores, or the like, may be used. Examples of the refractory porous material may include alumina (Al₂O₃), zeolite, activated carbon, charcoal, silica, and a mixture and a combination thereof.

Further, according to an embodiment of the present invention, a catalyst may not be used, but a porous material doped with specific metals or inorganic materials may be used. For example, a refractory porous material, which is doped with at least one metal component selected from Ag, Au, Na, Mg, Ca, Pt, Rh, Zn, Co, Cu, Rh, and the like, may be used. However, according to an embodiment of the present invention, it was confirmed that the use of a metal-doped porous material did not increase the FAME conversion. Further, when a porous material is doped with a metal component or the like, the amounts of C₁₂-C₂₀ hydrocarbon compounds, which are within the range of diesel, aromatic compounds, or the like, may somewhat increase due to cracking of oils.

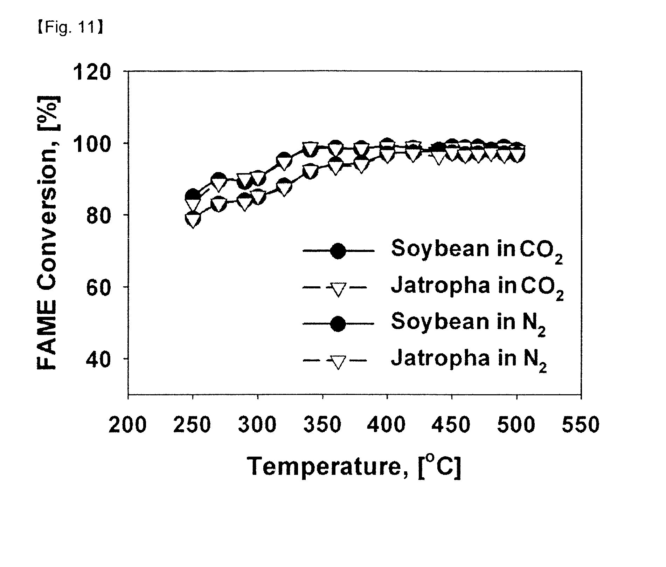

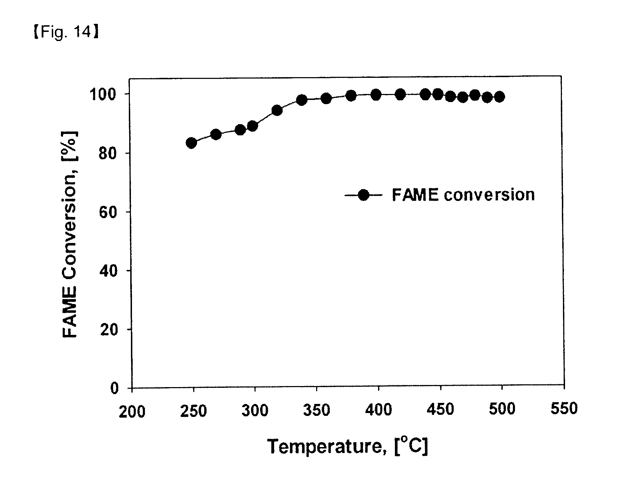

The transesterification reaction according to an embodiment of the present invention is characterized by the application of the minimum heat such that oils as reactants may reach activation energy by only heat energy without thermal cracking of the reactants. In this regard, the reaction temperature of the transesterification reaction according to an embodiment of the present invention, for example, the temperature of the reactor may be preferably 350 to 500°C. As may be confirmed in FIGS. 11 and 14 and descriptions thereof, the reaction temperature of the transesterification reaction according to an embodiment of the present invention may be extended to about 250°C while the conversion may be lowered or the reaction time may be lengthened. However, it is determined that the upper limit of the reaction temperature is preferably controlled to be lower than 550°C. For example, thermal cracking of the cooking oil was observed at 550°C.

Further, the transesterification reaction according to an embodiment of the present invention may be employed in the conditions of normal pressure, in which the conversion was excellent. From experimental examples by the present inventors, the transesterification reaction according to an embodiment of the present invention seems to be not limited by the pressure. For example, the reaction was possible even in the conditions of high pressure or reduced pressure. However, the normal pressure may be more advantageous in the view of process efficiency and costs. The reaction pressure according to an embodiment of the present invention may be, for example, 10 mmHg to 10 atm, preferably 0.5 to 5 atm, and more preferably 1 to 5 atm.

The transesterification reaction may be performed at the foregoing temperature and pressure conditions while the retention time is 0.1 to 5 minutes, 0.2 to 4 minutes, or 0.3 to 3 minutes. Particularly, the transesterification reaction according to an embodiment of the present invention may be performed for a retention time of 0.1 minutes or more in order to effectively increase the energy levels of the oils and the alcohols as reactants in the presence of the refractory porous material, and allows efficient evaporation and adsorption of the reactants. However, the retention time may be controlled to be 5 minutes or less to prevent the prolongation of the reaction time and the reduction in reactivity. Understandably, the retention time in the transesterification reaction according to an embodiment of the present invention may be appropriately selected depending on the design of the reactor structure. In an embodiment of the present invention, this reaction time is significantly reduced as compared with at least two hour, which is needed for pretreatment and main treatment processes when an acidic catalyst and a basic catalyst are used in the related art, and thus very excellent process efficiency may be achieved. Further, this retention time exhibits very excellent process efficiency as compared with a reaction time of about 5 to 20 minutes in a supercritical transesterification reaction that is known to be under development in the current research stage.

The transesterification reaction according to an embodiment of the present invention may be performed in a heterogeneous reaction of liquid and gas phases in the presence of the porous material. In the reaction conditions according to an embodiment of the present invention, the oils are determined to have a liquid phase, but not necessarily so. Since alcohols having a low melting point are in a gas phase at the corresponding conditions, the transesterification reaction according to an embodiment of the present invention seems to be performed in a manner in which gas-phase alcohols react with liquid-phase oils to generate gaseous FAMEs. Since triglyceride, which is a main component of the oils, is present in a liquid phase or gas phase, the triglyceride is easily adsorbed on the porous material, and its energy level may be increased by a heat source. The activation energy of the transesterification reaction may be sufficiently reached by the heat source.

The catalyst-free continuous type reaction according to an embodiment of the present invention may produce FAMEs even without purge gas, but the purge gas may be used to control the retention time of the reaction and induce a smooth continuous process. As the purge gas, an inactive gas is generally used, and for example, at least one of nitrogen (N₂), argon (Ar), carbon dioxide (CO₂), or the like may be used. This purge gas may be supplied into the reactor together with the reactants such as oils and the like.

According to an embodiment of the present invention, carbon dioxide (CO₂) or a gas containing at least carbon dioxide may be preferable as the purge gas. As a result of the transesterification reaction according to an embodiment of the present invention, a coking phenomenon may occur in the porous material. This coking phenomenon may disturb the continuous transesterification process according to an embodiment of the present invention. However, carbon dioxide used as the purge gas significantly reduces the coking phenomenon. When carbon dioxide is used as the purge gas, the conversion of fatty acid alkyl esters may be somewhat improved in the catalyst-free transesterification reaction according to an embodiment of the present invention. As may be confirmed in FIG. 11, the use of carbon dioxide as a purge gas may increase the FAME conversion by about 3∼4% as compared with the use of the other purge gas.

In the method for producing biodiesel according to an embodiment of the present invention, the transesterification reaction may be performed in a continuous process. In the process for producing biodiesel in a continuous type according to an embodiment of the present invention, the transesterification reaction may be continuously performed when oils and aliphatic alcohols are continuously supplied into a reactor in which a refractory porous material is present. In most cases, the reactor is previously charged with the refractory porous material while the refractory porous material is immobilized, but in some cases, the refractory porous material may be continuously supplied into the reactor depending on the kind of the reactor.

According to an embodiment of the present invention, a biomass having pores itself may be used as a porous material. An example thereof is disclosed in Koran Patent Application No. 2011-0101961, filled by the present inventors, the disclosure of which is incorporated herein by reference.

According to an embodiment of the present invention, since only oils and aliphatic alcohols react with each other without catalysts in the presence of the foregoing porous material to produce biodiesel, the obtained biodiesel may have minimized impurities and high purity. Here, the conversion may also have a significantly improved degree as compared with the related art.

The fatty acid alkyl esters generated through the transesterification reaction according to an embodiment of the present invention may contain an aliphatic moiety having 10 to 24 carbon atoms, preferably 12 to 22 carbon atoms, and more preferably 14 to 20 carbon atoms.

The transesterification reaction according to an embodiment of the present invention is shown in Reaction mechanism 1 below.

### [Reaction mechanism 1]

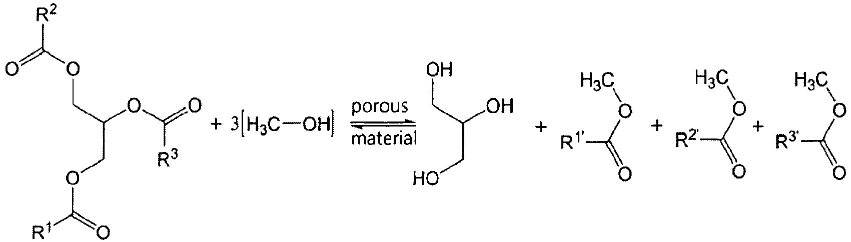

wherein, R¹, R², R³, R^{1'}, R^{2'} and R^{3'} are the same as or different from each other and each may be a C₄-C₃₈ aliphatic hydrocarbon group, preferably a C₄-C₂₄ aliphatic hydrocarbon group, and more preferably a C₁₂-C₂₀ aliphatic hydrocarbon group.

Hereinafter, the process for producing biodiesel according to an embodiment of the present invention will be described in detail with reference to the accompanying drawings such that the present invention may be easily implemented by a person having ordinary skill in the art.

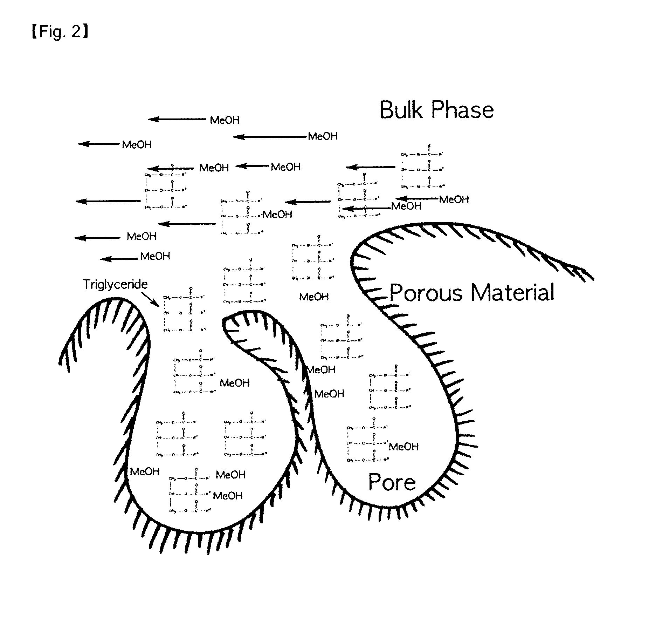

First, as shown in FIG. 2, the process for producing biodiesel according to an embodiment of the present invention is characterized by using a porous material.

As shown in FIG. 3, the kinetic energy of evaporated MeOH which is present in pores or on a bulk phase increases due to a high temperature. MeOH having improved activity collides with triglyceride adsorbed on or contained in the pores to induce a transesterification reaction. FAMEs and glycerin, which are reaction products, are released from a reactor while they are in a gas phase due to the reaction temperature. These gas-phase reaction products may become high-purity FAMEs and glycerin through only condensation thereof. It is known that glycerin may be easily separated from FAMEs.

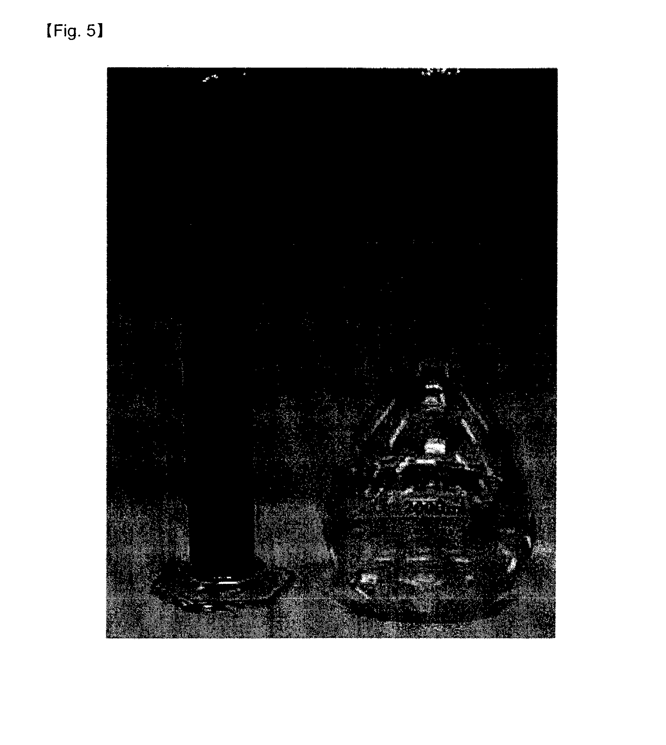

An example of a mixture of FAME and glycerin (left part) and an example of glycerin-removed FAME (right part), which are prepared according to an embodiment of the present invention, are comparatively shown in FIG. 5. The two flasks of biodiesel shown in FIG. 5 were obtained by using activated alumina (Al₂O₃), and may be confirmed to exhibit an FAME conversion of 99% at 400°C.

According to an embodiment of the present invention, the retention time of reactants supplied into the reactor for a continuous process does not exceed even one minute. According to an embodiment of the present invention, the transesterification reaction is completed within one minute to generate gas-phase products, which are then simply collected and purified to obtain FAME. As described above, the fast reaction rate of the transesterification reaction according to an embodiment of the present invention enables the design of a process for producing biodiesel in a continuous type. Since the retention time is determined depending on amounts of purge gas and MeOH, the reaction rate may be controlled by the purge gas.

In addition, the optimization of the catalyst-free continuous type transesterification reaction according to an embodiment of the present invention depends greatly on the boiling point of triglyceride and the boiling point of FAMEs.

According to an embodiment of the present invention, the reaction temperature of the process for producing biodiesel is designed such that oils, in other words, triglyceride receives a heat energy which is not smaller than an activation energy necessary for the reaction but does not allow thermal cracking, and such that FAMEs are present in a gas phase at the corresponding reaction temperature. Here, the oils are in a liquid phase, and alcohols having a low boiling point may be in a gas phase.

In order to obtain an appropriate reaction temperature in the process for producing biodiesel according to an embodiment of the present invention, the thermo-gravimetric analysis (TGA) was conducted as shown in FIG. 6. The thermo-gram shown in FIG. 6 was obtained by using a thermo-gravimetric analyzer by the NETZSCH Inc. Experiment was conducted from 20 to 1,000°C at a heating rate of 100 °C/min at the Ar atmosphere. FIG. 6 shows the mass change depending on the temperature and differential thermo-gram (DTG) obtained by primary differentiation of the mass change.

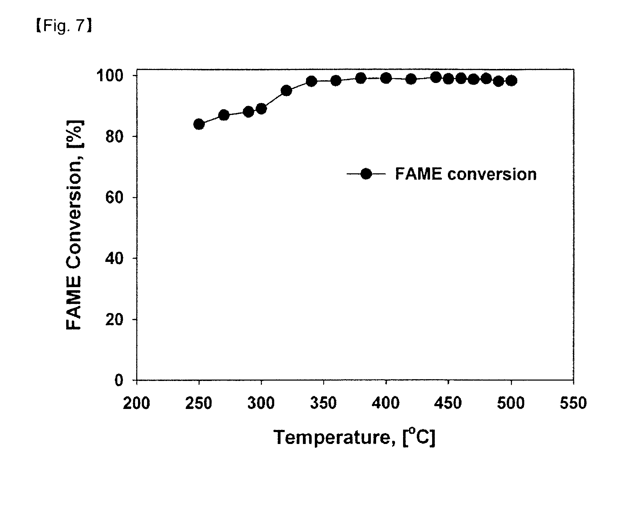

As shown in FIG. 6, triglyceride of the soybean oil has a boiling point of about 405.2°C, and its mass change reaches the maximum value at 452°C as seen from the DTG curve. Therefore, considering the results of FIG. 7, the reaction temperature of a biodiesel conversion process using triglyceride of the soybean oil may be preferably about 350∼400°C or 350-450 °C. When the reaction temperature is excessively increased, triglyceride may be unnecessarily evaporated and thus its evaporation latent heat may induce an energy loss. As described above, the upper limit of the reaction time is controlled to be lower than 550°C and preferably not higher than 500°C such that thermal cracking does not occur.

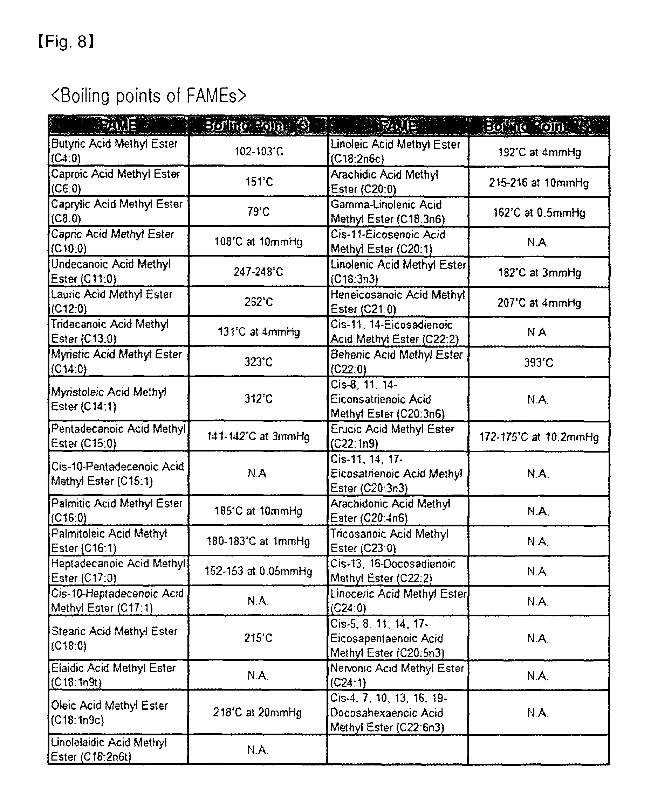

Further, in order to reduce the energy loss due to condensation of FAMEs obtained through an embodiment of the present invention and the evaporation latent heat of triglyceride, the reaction temperature is preferably 350°C or higher. This will be easily understood from boiling points of representative FAMEs shown in FIG. 8. Fatty acids of most oils shown in FIG. 8 have 14 to 20 carbon atoms (C₁₄-C₂₀). In order to obtain C₁₄-C₂₀ FAMEs, which are generated from the reaction according to an embodiment of the present invention, in a gas phase without a condensation procedure, the reaction temperature is preferably 350 to 500°C and more preferably 350 to 450°C. Considering that the boiling point of MeOH is 65°C, gas-phase FAMEs may be easily separated. The reaction temperature may be preferably 350 to 500°C and more preferably 350 to 450°C since energy may be saved in a purification process. Further, as summarized in FIG. 8, the boiling point of arachidic acid methyl ester is about 215 to 216°C, but this value is obtained at 10 mmHg but not normal pressure. Therefore, the biodiesel conversion process requires a temperature of 50°C or higher. However, the excessive increase in the reaction temperature may maximize the energy loss due to the evaporation latent heat of triglyceride, which is not recommendable.

An example of the FAME conversion depending on the temperature change in the transesterification reaction is shown in FIG. 7. The reaction was performed at atmospheric pressure. Activated alumina (Al₂O₃) was used as a porous material. The weight ratio of MeOH to oil was about 0.2:1. As shown in FIG. 7, a high FAME conversion of 98% to 99% could be obtained at a reaction temperature of 350°C or higher. In addition, as shown in FIG. 7, the transesterification reaction according to an embodiment of the present invention is possible even at a temperature of 350°C or lower. In this case, however, the FAME conversion may be somewhat decreased to 90% or lower, and the reaction time may be somewhat lengthened.

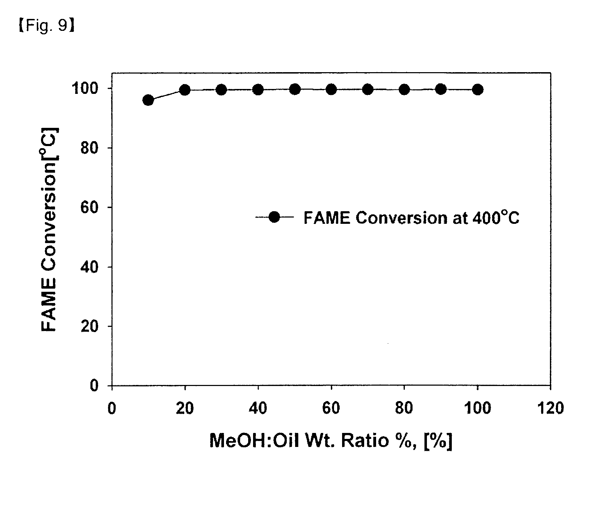

Meanwhile, in the catalyst-free continuous type transesterification process according to an embodiment of the present invention, the reactants are triglyceride and MeOH. The weight ratio of MeOH to triglyceride as the reactants is preferably 0.1:1. This value is calculated in the view of stoichiometry and may be confirmed in FIG. 9.

For optimization of oils, that is, triglyceride, and MeOH in the process according to an embodiment of the present invention, experiments were conducted for various weight ratios of MeOH to triglyceride. In the experiment in FIG. 9, a transesterification reaction for biodiesel was performed at a temperature of 400°C and at atmospheric pressure, and charcoal was used as a porous material. As shown in FIG. 9, it may be seen that the FAME conversion was very excellent at a MeOH percentage of preferably 20% or more. Even when another porous material according to an embodiment of the present invention, for example, activated alumina (Al₂O₃) was used, nearly similar results as shown in FIG. 9 were obtained.

FIG. 11 is a graph showing that the FAME conversion was increased when carbon dioxide was used as a purge gas. The experiment for FIG. 11 was conducted at atmospheric pressure, activated alumina as a porous material and methanol as alcohol were used.

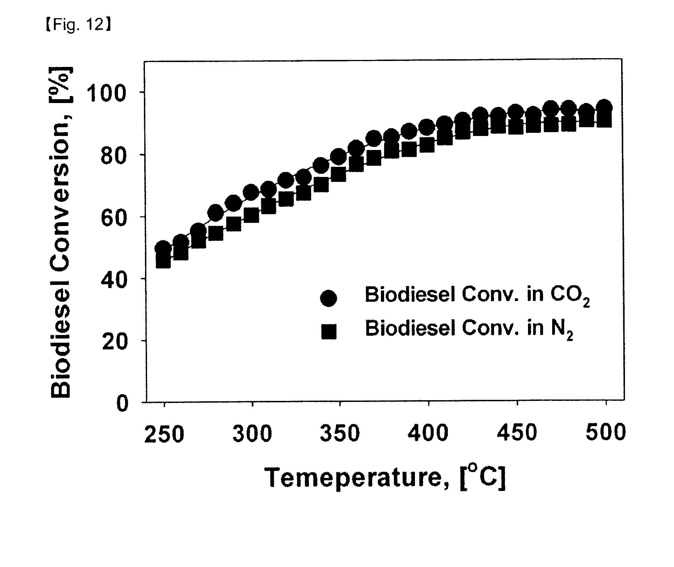

In addition, FIG. 12 is a graph showing the FAME conversion according to the purge gas while 100% of fatty acid was used for the reaction. In the experiment for FIG. 12, activated alumina as a porous material, methanol as alcohol, and oleic acid as oils were used at atmospheric pressure. It was seen from FIG. 12 that, even when 100% fatty acid having an acid value of approximately 200 was used in this experiment, the FAME conversion was about 90% or more at a temperature of about 400°C or higher.

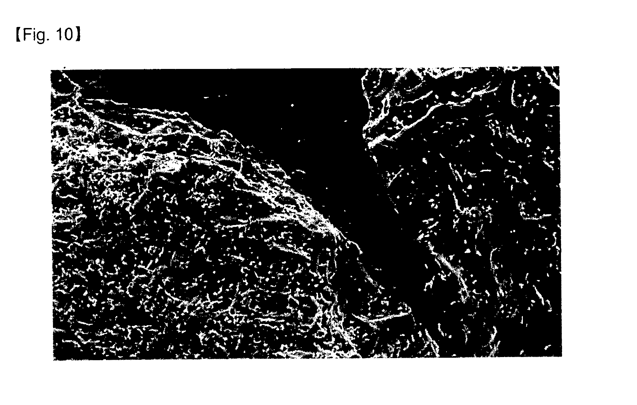

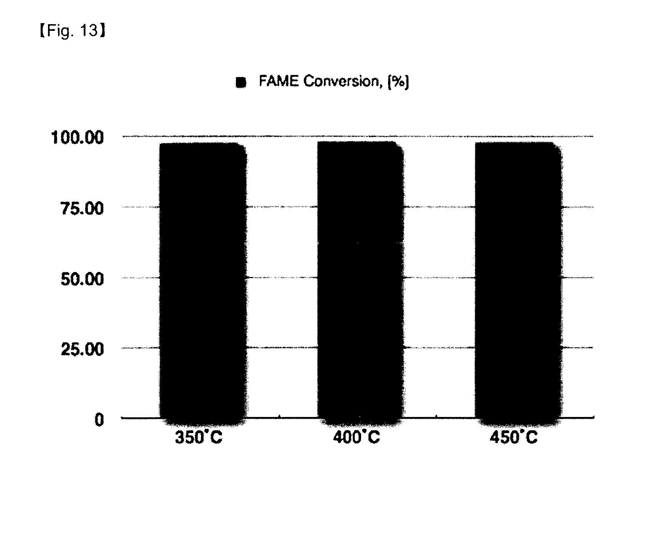

According to an embodiment of the present invention, the reaction results when charcoal was used instead of activated alumina (Al₂O₃), as a porous material, are shown in FIG. 13. Here, the weight ratio of MeOH to oil was 0.2:1 and the experiment temperatures were 350°C, 400°C, and 450°C. The porous structure of charcoal may be confirmed from the SEM image of FIG. 10. Meanwhile, it may be confirmed from FIG. 9 that a high conversion was obtained by the use of the charcoal. According to an embodiment of the present invention, porous materials such as silica and zeolite other than charcoal may be used in the conversion into FAME.

The foregoing method for producing biodiesel according to an embodiment of the present invention overcame disadvantages of the transesterification process for producing biodiesel of the related art, and seems to overcome drawbacks of a transesterification reaction under active development in the current R&D stage.

According to an embodiment of the present invention, biodiesel may be obtained through a continuous process.

According to an embodiment of the present invention, a biodiesel conversion process may be effectively performed regardless of the amount of free fatty acids, unlike the process of the related art in which the process and the process operation are changed depending on the amount of free fatty acids (FFAs) present in oils.

In addition, according to an embodiment of the present invention, since the catalyst used in the transesterification reaction process is not used, the biodiesel conversion process may be constructed in a manner in which pretreatment and main treatment are integrated. Further, according to an embodiment of the present invention, the integration type of pretreatment/main treatment process may fundamentally prevent the generation of waste water and the loss of biodiesel (FAMEs).

Further, an embodiment of the present invention is characterized in that the reaction is performed at normal pressure, unlike a transesterification process through a high-temperature/high-pressure supercritical reaction which is currently under R & D.

According to an embodiment of the present invention, carbon dioxide is used in the process, which shows a definite contrast to the commercialized transesterification process of the related art and the transesterification process under R & D. This is an environmentally friendly process, which corresponds to the effect according to an embodiment of the present invention.

Further, according to an embodiment of the present invention, a definite energy saving effect may be exhibited since the biodiesel may be more quickly produced as compared with the commercial process of the related art. Particularly, since biodiesel (FAMEs) is produced from only methanol and oils as reactants, the costs for distilling purification may be expected to be remarkably saved.

The conversion of fatty acid alkyl esters which are generated from the transesterification reaction performed in the presence of a refractory porous material according to an embodiment of the present invention may be 90% or higher, preferably 95% or higher, and more preferably 98% or higher.

Contents other than the above descriptions may be added or deleted as necessary, and thus are not particularly limited herein.

Hereinafter, although preferable examples are provided to help understandings of the present invention, the following examples are provided merely to illustrate the present invention, but the scope of the present invention is not limited to the following examples.

### Examples 1-16

At conditions as shown in Table 1 below, oils and C₁-C₁₂ aliphatic alcohols were subjected to a transesterification reaction in a continuous type in the presence of a refractory porous material, and the thus generated fatty acid alkyl esters and glycerol were collected to produce biodiesel. Particularly, soybean oil for Examples 1 to 14, jatropha oil for Example 15, and extracted lipid from beef tallow and lard for Example 16 were used as the oils.

Here, the conversion of the generated fatty acid methyl ester (FAME) was measured as follows.

### <FAME conversion>

The FAME conversion was obtained by using an analysis value through GC/MS. The ASTM D6751 or EN14214 standard is acceptable for analysis of biodiesel. Particularly, the FAME yield was obtained by using EN14103 (determining the ester and linoleic acid methyl ester content). Besides, EN14106/ASTM D6584 was used to determine glycerin and mono-, di-, and triglyceride contents.

**(Table 1)**

| Classification | Oils | Alcohols | Porousmaterial | Weight ratio of oil to alcohol | Charing gas | Reactiontemperature (°C) | Reaction pressure (mmHg) | Retention time (min) | FAME conversion (%) |
|---|---|---|---|---|---|---|---|---|---|
| Example 1 | Soybean oil | MeOH | Al₂O₃ | 1:0.2 | CO₂ | 400 | 760 | 1 | 98 |
| Example 2 | Soybean oil | MeOH | Al₂O₃ | 1:0.2 | CO₂ | 350 | 760 | 1 | 99 |
| Example 3 | Soybean oil | MeOH | Al₂O₃ | 1:0.2 | CO₂ | 450 | 760 | 1 | 99 |
| Example 4 | Soybean oil | MeOH | Al₂O₃ | 1:0.1 | CO₂ | 400 | 760 | 1 | 95 |
| Example 5 | Soybean oil | MeOH | Al₂O₃ | 1:0.4 | CO₂ | 400 | 760 | 1 | 99 |
| Example 6 | Soybean oil | MeOH | Al₂O₃ | 1:0.6 | CO₂ | 400 | 760 | 1 | 99 |
| Example 7 | Soybean oil | MeOH | Al₂O₃ | 1:0.2 | CO₂ | 400 | 1,520 | 1 | 99 |
| Example 8 | Soybean oil | MeOH | Al₂O₃ | 1:0.2 | CO₂ | 400 | 10 | 1 | 99 |
| Example 9 | Soybean oil | MeOH | Al₂O₃ | 1:0.2 | CO₂ | 400 | 760 | 0.1 | 99 |
| Example 10 | Soybean oil | MeOH | Al₂O₃ | 1:0.2 | CO₂ | 400 | 760 | 2 | 99 |
| Example 11 | Soybean oil | MeOH | Al₂O₃ | 1:0.2 | N₂ | 400 | 760 | 1 | 95 |
| Example 12 | Soybean oil | MeOH | Charcoal | 1:0.2 | CO₂ | 400 | 760 | 1 | 99 |
| Example 13 | Soybean oil | MeOH | MgO-CaO/ Al₂O₃ | 1:0.2 | CO₂ | 400 | 760 | 1 | 98 |
| Example 14 | Soybean oil | EtOH | Al₂O₃ | 1:0.2 | CO₂ | 400 | 760 | 1 | 99 |
| Example 15 | Jtropha oil | MeOH | Al₂O₃ | 1:0.2 | CO₂ | 400 | 760 | 1 | 98 |
| Example 16 | beef tallow/lard | MeOH | Charcoal | 1:0.2 | CO₂ | 400 | 760 | 1 | 99 |

### Comparative Example 1

Following the related art, biodiesel was produced by performing a transesterification reaction at a reaction temperature of 65°C for 30 hours while H₂SO₄ was used as an acidic catalyst. Here, the conversion of the generated fatty acid methyl esters (FAMEs) was 94%.

### Comparative Example 2

Following the related art, biodiesel was produced by performing a transesterification reaction at a reaction temperature of 65°C for 2 hours while NaOH was used as a basic catalyst. Here, the conversion of the generated fatty acid methyl esters (FAMEs) was 94%.

### Comparative Example 3

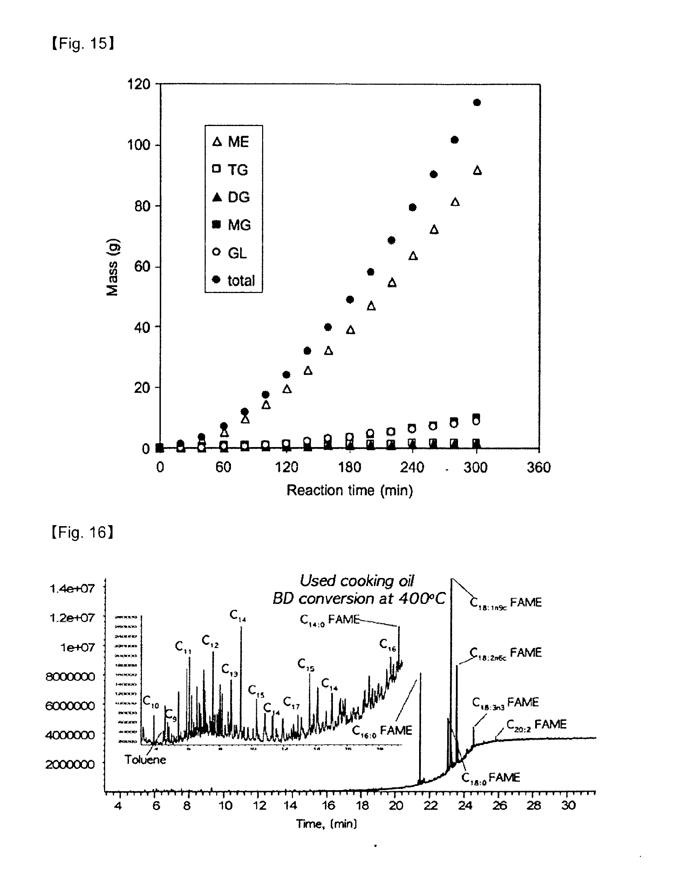

Biodiesel was produced by performing a transesterification reaction through the same method as in Example 1 except that a catalyst-free thermodynamic conversion method was applied for a reaction time of 300 minutes or more by using bubbling instead of a porous filler while not using the acidic or basic catalyst of the related art. Here, the conversion of the generated fatty acid methyl esters (FAMEs) was 91%. In this regard, an example of the results is shown in FIG. 15. In FIG. 15, ME represents methanol, TG represents triglyceride, DG represents diglyceride, and MG represents monoglyceride.

As shown in Table 1, in Examples 1 to 16 in which, following the present invention, a transesterification reaction was performed in the presence of a refractory porous material while acidic and basic catalysts were not used, the reaction time was about 1 minute and the conversion was 95% or more and more favorably 99% or more, resulting in a high conversion in a short time for each of the examples. While, in Examples 1 and 2 in which, following the related art, acidic and basic catalysts were used, the conversion was merely about 94% for each of the examples. In the case of Example 3 in which a thermo-chemical conversion reaction was performed without charging with a porous material, the conversion was merely 91% even for a long reaction time of 300 minutes or more.

Meanwhile, FIG. 16 shows a mass chromatogram obtained by performing gas chromatography-mass spectroscopy (GC-MS) on products, which were prepared from a transesterification reaction using beef oil and pork oil in Example 16. As confirmed in FIG. 16, in Example 16 in which, following the present invention, the transesterification reaction was performed by using beef tallow and lard while not using separate acidic and basic catalysts, the conversion into biodiesel (fatty acid methyl esters, FAMEs) was effectively achieved without the detection of fatty acids corresponding to initial components.

While this invention has been described in connection with what is presently considered to be practical embodiments, it is to be understood that the invention is not limited to the disclosed embodiments, but, on the contrary, is intended to cover various modifications and equivalent arrangements included within the spirit and scope of the appended claims.

## Claims

1. A method for producing biodiesel, the method comprising generating fatty acid alkyl esters and glycerol by a transesterification reaction of oil and an aliphatic alcohol in a presence of a refractory porous material.

2. The method of claim 1, wherein a temperature of the transesterification reaction is controlled such that the transesterification reaction proceeds in a presence of the porous material, by raising energy levels of the oil and the aliphatic alcohol through a supply of heat energy; and
wherein the temperature of the transesterification reaction is controlled such that the fatty acid alkyl esters are generated in a gas phase and thermal cracking of the oil is not generated.

3. The method of claim 2, wherein the temperature of the transesterification reaction is 350 to 500°C.

4. The method of claim 3, wherein the weight ratio of the oil to the alcohol is 1:0.05 to 1:1.

5. The method of claim 4, wherein a retention time of the transesterification reaction is 0.1 to 5 minutes.

6. The method of claim 1, wherein the transesterification reaction is performed in a gas atmosphere of at least one of nitrogen, argon, or carbon dioxide.

7. The method of claim 1, wherein a pressure of the transesterification reaction is 10 mmHg to 10 atm.

8. The method of claim 1, wherein the transesterification reaction is performed in a heterogeneous phase reaction.

9. The method of claim 1, wherein the transesterification reaction is performed in a continuous process.

10. The method of claim 1, wherein the refractory porous material is at least one of alumina (Al₂O₃), zeolite, activated carbon, charcoal, or silica.

11. The method of claim 1, wherein the refractory porous material includes a mesoporous material, a macroporous material, or both thereof.

12. A method for producing biodiesel, the method comprising:
inducing a transesterification reaction between oil and an aliphatic alcohol by continuously supplying the oil and the aliphatic alcohol into a reactor charged with a refractory porous material; and
collecting gas-phase fatty acid alkyl esters and glycerol, which are generated by the transesterification reaction.

13. The method of claim 12, wherein in the inducing of the transesterification reaction, a purge gas is continuously supplied together with the oil and the aliphatic alcohol, the purge gas being at least one of nitrogen, argon, or carbon dioxide.

14. The method of claim 12, wherein a temperature of the reactor is controlled such that the transesterification reaction proceeds in a presence of a porous material, by raising energy levels of the oil and the aliphatic alcohol through a supply of heat energy; and
wherein the temperature of the transesterification reaction is controlled such that the fatty acid alkyl esters are generated in a gas phase and thermal cracking of the oil is not generated.
